# FASCICULE DE BREVET EUROPEEN

(11) **EP 1 941 014 B1**
(45) Date de publication et mention de la délivrance du brevet: **06.11.2013**
(21) Numéro de dépôt: 06808164.5
(22) Date de dépôt: 19.09.2006
(51) Int. Cl.: C11C 3/04, C07C 67/03

(54) **PROCEDE AMELIORE DE FABRICATION D'ESTERS ETHYLIQUES A PARTIR DE CORPS GRAS D'ORIGINE NATURELLE**
VERBESSERTES VERFAHREN ZUR HERSTELLUNG VON ETHYLESTERN AUS NATÜRLICHEN FETTEN
IMPROVED METHOD FOR MAKING ETHYL ESTERS FROM NATURAL FATS

(30) Priorité: 21.09.2005 FR 0509734
(43) Date de publication de la demande: 09.07.2008
(73) Titulaire: Institut Français du Pétrole, 92852 Rueil Malmaison Cedex (FR)
(72) Inventeur: HILLION, Gérard, F-95220 Herblay (FR); DELFORT, Bruno, F-75005 Paris (FR)
(86) Numéro de dépôt international: PCT/FR2006/002142
(87) Numéro de publication internationale: WO 2007/034068

(56) Documents cités:
- DE-A1- 4 238 195
- US-A- 5 354 878
- US-A- 5 434 279
- US-A- 5 514 820
- US-A1- 2003 004 363

## Description

La présente invention concerne la fabrication d'esters éthyliques d'acides gras utilisables comme substituts du gazole, à partir d'huiles ou de graisses naturelles, végétales ou animales, ou d'autres mélanges de glycérides.

Elle a plus particulièrement pour objet un procédé amélioré de transestérification permettant, à partir d'huiles ou de graisses naturelles, végétales ou animales, ou d'autres mélanges de glycérides, l'obtention quasi-quantitative d'èsters éthyliques d'acides gras.

La réaction de transestérification par le méthanol et l'éthanol est bien connue de l'homme du métier. Elle utilise le plus souvent des catalyseurs homogènes, par exemple des catalyseurs acides (acides sulfoniques, acide sulfurique etc.), comme décrit notamment dans le brevet US-A-4 695 411, des composés métalliques divers, par exemple des sels métalliques comme des sels de titane, de zinc, de magnésium, d'étain, d'antimoine ou de plomb, ces composés métalliques pouvant être sous la forme d'alcoolates, de dérivés alkyles ou d'oxydes. De préférence, on utilise plus particulièrement, de par leur grande réactivité, des catalyseurs basiques homogènes du type NaOH, KOH ou LiOH en solution dans le méthanol, ou directement des alcoolates de ces mêmes métaux, ou encore certains carbonates comme par exemple le carbonate de potassium et le carbonate de sodium, comme cités par Freedman B. et al : JAOCS 61 n°10, p.1638 ; par Pryde E.H., "Vegetable Oil Fuels", Proc. Int. Conf., Fargo, ND, 1982, pp. 117-122 ; et dans le brevet US-A-2 383 602.

. La transestérification en présence de méthanol est généralement réalisée en une seule étape de catalyse dans le cas d'une réaction conduite en batch ou au minimum en deux étapes de catalyse lorsque l'on opère en continu par l'intermédiaire de réacteurs à débordement.

Les procédés d'éthanolyse décrits sont peu nombreux et beaucoup moins performants que ceux qui utilisent le méthanol. En effet, avec l'éthanol et en utilisant un même ratio molaire alcool/huile qu'avec le méthanol, il est impossible d'obtenir naturellement une séparation du glycérol formé (voir notamment le brevet US-A-2 383 602).

Le pouvoir solvant de l'éthanol étant bien supérieur à celui du méthanol, ceci a pour conséquence de rendre soluble la glycérine formée pendant la réaction. La conversion en esters s'en trouve pénalisée, ce qui empêche d'obtenir en une seule étape de réaction une conversion élevée.

Une seconde étape de transestérification est donc nécessaire après qu'on a éliminé du milieu réactionnel la glycérine formée,

La distillation partielle d'éthanol ou l'ajout d'une certaine quantité d'eau ou d'un tiers solvant tel qu'un hydrocarbure, par exemple du n-heptane, permet de diminuer la solubilité de la glycérine et d'en éliminer une quantité suffisante, ce qui permet d'atteindre en deuxième étape de catalyse une conversion élevée. Une autre alternative permettant d'atteindre des conversions élevées consiste à distiller dans certaines conditions et sous pression réduite les esters éthyliques fabriqués au cours de la première étape de transestérification.

Le document DE 42 38 195 décrit un procédé de production d'esters méthyliques de colza comprenant deux étapes de transestérification successives, suivies chacune de deux étapes de séparation de la glycérine.

Le document US-5,354,878 décrit un procédé de transestérification de méthanol et d'acides gras, mis en oeuvre à pression ambiante et à une température inférieure à 100°C, en présence d'un catalyseur alcalin, le procédé étant réalisé de façon continu en une succession de deux étapes de transestérification, chacune étant répétée une deuxième fois.

Le document US-5,514,820 décrit quant à lui un procédé continu de production d'esters méthyliques, à des températures allant jusqu'à 100°C, à des pressions allant jusqu'à 10 bars, par réaction de triglycérides contenant moins de 1% d'acides gras libres avec du méthanol, le procédé mettant en oeuvre au moins deux étapes dans un réacteur tubulaire, en présence d'un catalyseur homogène alcalin. La glycérine formée est retirée du mélange réactionnel après chaque étape, par un séparateur statique horizontal.

On a maintenant découvert, de manière inattendue, que l'on pouvait réaliser l'éthanolyse d'une huile, d'une graisse, ou de tout autre mélange de glycérides, en deux étapes de catalyse en utilisant un catalyseur basique soluble, tout en obtenant une décantation naturelle de la glycérine après la première étape de transestérification, sans avoir recours à une distillation de l'excès d'alcool ou à un ajout d'eau ou d'un tiers solvant.

Ainsi, l'invention propose un procédé de fabrication d'esters éthyliques d'acides gras à partir d'huiles ou de graisses végétales ou animales ou d'autres mélanges de glycérides qui comprend la succession d'étapes suivantes :
- une étape (a) dans laquelle on transestérifie l'huile, la graisse, ou le mélange de glycérides dont l'indice d'acide est au plus de 2 par de l'éthanol dont la teneur en eau est comprise entre 3000 et 5000 ppm en utilisant un catalyseur soluble, ou le devenant au cours de la réaction ;
- une étape (b) de décantation et d'élimination de la glycérine formée ;
- une étape (c) dans laquelle on réalise une deuxième réaction de transestérification de façon à obtenir un produit dont la teneur en esters est d'au moins 97 % en masse ;
- une étape (d) dans laquelle on réalise une évaporation de l'excès d'éthanol en présence du catalyseur, par emploi d'un évaporateur de type film tombant, à une température inférieure à 120°C et un temps de séjour inférieur à 1 minute, l'éthanol obtenu étant alors pratiquement anhydre ;
- une étape (e) dans laquelle l'ester subit une purification par des séquences de lavage à l'eau ; et
- une étape (f) dans laquelle le mélange d'esters est séché sous pression réduite.

Dans le procédé de l'invention, en répartissant judicieusement la stoechiométrie en éthanol sur les deux étapes (a) et (c) de transestérification et en utilisant comme catalyseur soit un alcoolate alcalin obtenu par dissolution dans un alcool (méthanol ou éthanol) d'un hydroxyde de sodium, de potassium ou de lithium, soit une solution méthanolique de méthylate de sodium disponible commercialement, on obtient aisément à l'issue de la première étape de catalyse [étape (a)] une conversion en esters fabriqués supérieure ou égale à 90 %. Il est alors possible, après élimination de la glycérine formée, d'effectuer La seconde étape de catalyse [étape (c)] est alors effectuée après l'ajout d'un appoint d'éthanol et de catalyseur. La conversion en esters éthyliques ainsi obtenue est alors suffisante pour atteindre la qualité demandée à un mélange d'esters à usage carburant.

L'utilisation comme catalyseur, dans les étapes (a) et (c), d'un alcoolate de métal alcalin, par exemple de l'éthylate ou du méthylate de sodium ou de potassium, offre l'avantage d'obtenir de l'éthanol pratiquement anhydre en fin de deuxième étape de catalyse. L'alcoolate joue en effet le rôle d'un desséchant, comme le montre l'équation chimique d'hydrolyse de l'alcoolate par l'eau (l'eau provenant essentiellement des charges : huile et éthanol). Cette réaction est stoechiométrique et, dans le cas du méthylate, elle s'écrit comme suit :

MOCH₃ + H₂O → CH₃OH + MOH

(où M représente le métal alcalin). -Ceci est un moyen élégant d'éliminer physiquement l'eau du milieu réactionnel.

L'excès d'éthanol présent dans le milieu réactionnel que l'on évapore après la seconde étape de catalyse [étape (c)], est pratiquement anhydre et présente l'avantage de pouvoir être recyclé sans qu'on ait recours à une étape de rectification. Cette évaporation est conduite dans des conditions telles qu'on évite une rétro-réaction qui consiste en la réaction de la glycérine avec les esters éthyliques fabriqués pour reformer des glycérides et de l'éthanol. La température et le temps de séjour sont les deux principaux paramètres qui influent sur la vitesse de cette réaction inverse.

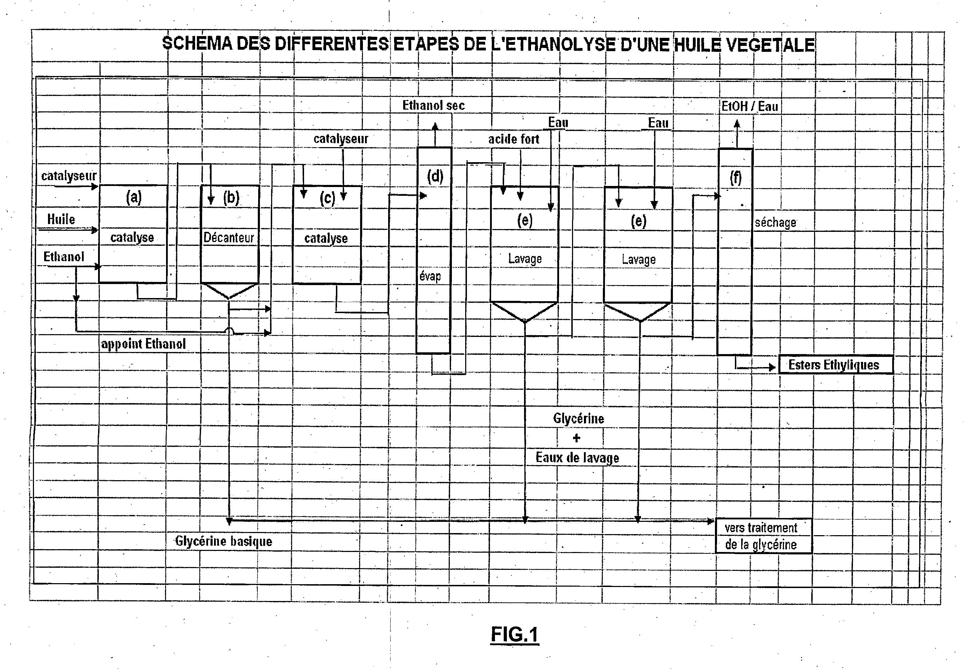

Les différentes étapes du procédé de l'invention sont décrites plus en détail ci-après, en liaison avec la figure annexée.

### Étape (a)

L'huile, la graisse, végétale ou animale, ou le mélange de glycérides de départ présente un indice d'acide d'au plus 2. On utilise de l'éthanol dont la teneur en eau est comprise entre 3000 et 5000 ppm, en utilisant une stoechiométrie éthanol/huile telle qu'on obtient une conversion en esters fabriqués supérieure ou égale à 90 %. Cette stoechiométrie éthanol/huile est généralement comprise entre 1, 3 et 2, de préférence entre 1,6 et 1,8.

L'intérêt de cette étape dans le procédé de l'invention est que sa mise en oeuvre permet, dans l'étape suivante (b), une décantation naturelle de la glycérine, sans qu'on ait recours à une opération d'évaporation de l'excès d'éthanol.

Le catalyseur utilisé est un catalyseur basique homogène, c'est-à-dire un catalyseur soluble, ou qui le devient au cours de la réaction. Il peut être obtenu par exemple par dissolution d'une base forte dans un alcool (méthanol ou éthanol), ou encore à partir d'un alcoolate de métal alcalin, qui peut être par exemple un éthylate ou un méthylate de sodium, ou à partir d'un composé métallique de type alcoolate, alkyle et/ou oxyde. Le méthylate de sodium est préféré car il présente l'avantage d'être un produit bon marché, disponible industriellement en solution à 30 % dans le méthanol.

La température de réaction est généralement comprise entre 20 et 100°C, de préférence entre 40 et 80°C.

En mode batch, le temps de réaction qui permet d'atteindre l'équilibre thermo-dynamique est généralement compris entre 40 et 160 minutes.

On notera que si l'on utilise comme catalyseur un méthylate de métal alcalin en solution dans le méthanol, le mélange d'esters obtenus contiendra une certaine proportion d'esters méthyliques, en général entre 10 et 15 % en masse.

### Étape (b)

La décantation naturelle de la glycérine est obtenue dès l'arrêt de l'agitation et à une température du milieu comprise par exemple entre 40 et 60°C. La glycérine contenue dans la phase inférieure est ensuite éliminée par soutirage.

### Étape (c)

Cette deuxième étape de transestérification est réalisée après ajout d'une nouvelle quantité de catalyseur alcalin et d'une quantité d'éthanol correspondant à une stoechiométrie éthanol/huile de départ comprise en général entre 0,3 et 1, de préférence entre 0,5 et 0,7.

La température de catalyse est du même ordre de grandeur que celle de l'étape (a), avec un temps de catalyse compris entre 20 et 45 minutes.

Par cette étape (c), on obtient un produit dont la teneur en esters est d'au moins 97 % en masse.

### Étape (d)

Dans cette étape, l'élimination de l'excès d'éthanol du milieu réactionnel se fait en général par évaporation en présence du catalyseur. L'éthanol obtenu est pratiquement anhydre et peut être recyclé dans le procédé sans avoir à subir une rectification.

Les conditions d'évaporation doivent être adaptées afin d'éviter la rétro-réaction, qui consiste en ce que le glycérol formé réagit avec les esters éthyliques fabriqués, en reformant des glycérides partiels (mono- et diglycérides) ou totaux (triglycérides), reconstituant ainsi l'huile de départ.

Le temps de séjour et la température sont les deux principaux paramètres qui influent sur la vitesse de cette réaction inverse. On emploie un évaporateur de type film tombant pour réduire le temps de séjour. La température est inférieure à 120°C, de préférence inférieure à 100°C. Le temps de séjour est inférieur à 1 minute, de préférence inférieur à 30 secondes.

### Étape (e)

Dans cette étape, on purifie la phase esters en la débarrassant du catalyseur résiduel et de la glycérine soluble par une succession de lavages à l'eau.

Si nécessaire, on peut effectuer un premier lavage neutralisant qui élimine efficacement les traces de savons de sodium contenus dans la phase esters en utilisant un acide minéral fort, comme l'acide sulfurique, l'acide chlorhydrique ou l'acide phosphorique, puis on effectue un ou plusieurs lavage(s) à l'eau pure, selon l'efficacité d'élimination de l'excès d'acide fort.

### étapes (f)

On élimine les traces d'eau et d'éthanol par un séchage (évaporation sous pression réduite), de façon que le mélange d'esters réponde aux spécifications d'un Biodiesel concernant les deux critères (teneurs en eau et en éthanol) de la norme EN 14214.

Les exemples suivants illustrent le procédé de l'invention.

### Exemple 1

Dans un réacteur en verre équipé d'une vanne de fond, agité et chauffé à 70°C, on introduit 1000 g d'huile de colza raffinée de qualité alimentaire et 266 g d'éthanol contenant 3000 ppm d'eau, en respectant une stoechiométrie éthanol /huile de 1,7. Puis on introduit 10 g d'une solution méthanolique à 30 % de méthylate de sodium. On maintient l'agitation et la température de 70°C ± 2°C pendant 60 minutes (ceci constitue l'étape (a)).

Dans l'étape (b), on décante le milieu réactionnel à 60°C. 15 à 20 minutes après l'arrêt de l'agitation, on soutire 95 g d'une solution glycérineuse composée de glycérine, d'éthanol, d'esters éthyliques, de savons de sodium et d'alcoolates (mélange de méthylate, d'éthylate, et de glycérate) de sodium.

A l'issue de cette première réaction de transestérification, la composition du mélange est la suivante (en % masse) :
- Esters éthyliques et méthyliques : 91,5
- Triglycérides : 0,9
- Diglycérides : 2,6
- Monoglycérides : 3,7
- Stérols et esters de stérols : 1,3.

Dans l'étape (c), dans le même réacteur, on réalise une deuxième -étape de transestérification. Pour ce faire, on ajoute à la phase esters 93g d'éthanol contenant 3000 ppm d'eau, ce qui correspond à une stoechiométrie éthanol/huile de départ de 0,6, et 3,33 g de méthylate de sodium en solution à 30 % dans le méthanol. On maintient en milieu agité à 60°C pendant au moins 30 minutes.

Dans ces conditions de température, la glycérine formée reste soluble dans le milieu réactionnel.

A l'issue de cette deuxième réaction de transestérification, la composition du mélange est la suivante (en % masse) :
- esters éthyliques et méthyliques 97,5
- triglycérides : < 0,1
- diglycérides : 0,2
- monoglycérides : 0,7
- stérols et esters de stérols : 1,6.

Dans l'étape (d), on élimine la plus grande partie de l'excès d'éthanol du milieu réactionnel en alimentant en continu un évaporateur à film tombant.

Les conditions opératoires sont les suivantes:
- débit d'alimentation : 600 ml/heure,
- température de consigne du film tombant : 100°C,
- pression : 160 mm de Hg,
- temps de séjour moyen estimé entre 15 et 20 secondes.

Dans ces conditions, aucune rétro-réaction n'a été constatée. En partant d'une teneur initiale en éthanol d'environ 17 % dans le mélange d'esters, on obtient à l'issue de cette étape une teneur résiduelle en éthanol comprise entre 1,8 et 2 % en masse. La teneur en eau de l'éthanol distillé est inférieure à 400 ppm.

Dans l'étape (e), on purifie la phase d'esters éthyliques obtenue en effectuant une série de lavages à l'eau.

Dans le réacteur équipé d'une vanne de fond, on introduit la totalité du mélange réactionnel et on amène sa température à 60°C. On ajoute de 30 g d'eau désionisée, on agite 5 minutes et on décante 15 à 20 minutes. On soutire une phase aqueuse enrichie en alcool, glycérine et sels de sodium. On répète l'opération jusqu'à obtention d'une valeur de pH de la solution aqueuse comprise entre 7 et 8.

La composition du mélange d'esters obtenu, qui respecte la norme EN 14214 relative à un Biodiesel, est la suivante (en % masse) :
- esters éthyliques et-méthyliques : 97,5
- triglycérides : < 0,1
- diglycérides : 0,2
- monoglycérides : 0,7
- stérols et esters de stérols : 1,6.

On recueille au final 1023 g d'un mélange d'esters éthyliques et méthyliques qui contient 10,3 % d'esters méthyliques. La présence d'esters méthyliques provient de l'utilisation comme catalyseur de méthylate de sodium, la totalité du méthanol mis en jeu étant alors transformée en esters méthyliques.

### Exemple 2 (comparatif)

On reproduit à l'identique l'Exemple 1, si ce n'est que, dans l'étape (d), la température de consigne du film tombant est portée à 148°C.

La quantité résiduelle d'éthanol dans le mélange d'esters est inférieure à 0,3 %.

Dans ces conditions, on constate une rétro-réaction, comme le montre la composition du mélange obtenu (en % masse):
- esters éthyliques et méthyliques : 66,2
- triglycérides : 13,9
- diglycérides : 16,1
- monoglycérides : 2,2
- stérols et esters de stérols : 1,6.

### Exemple 3 (comparatif)

On reproduit à l'identique l'Exemple 1 jusqu'à l'étape (e) incluse.

L'étape (d), qui consiste à éliminer la plus grande partie de l'excès d'éthanol, est réalisée en batch dans le réacteur équipé d'une vanne de fond.

Les conditions opératoires sont : une température du mélange réactionnel de 100°C et une pression de 160 mm de Hg.

La durée de l'évaporation nécessaire pour atteindre une teneur résiduelle en éthanol de l'ordre de 1,5 % en masse est de 30 minutes.

Dans ces conditions, la rétro-réaction est effective au vu de la composition du mélange (en % masse) :
- esters éthyliques et et méthyliques 94,4
- triglycérides : 0,4
- diglycérides : 1,9
- monoglycérides : 1,7
- stérols et esters de stérols : 1,6.

Les résultats de cet exemple montrent que, malgré une température de 100°C, identique à celle mise en jeu dans l'Exemple 1, le temps de séjour est un paramètres important qui conditionne l'apparition de la rétro-réaction. Le film tombant apparaît comme l'outil préféré pour allier une température et un temps de séjour convenables.

## Revendications

1. Procédé de fabrication d'esters éthyliques d'acides gras à partir d'huiles ou de graisses végétales ou animales ou d'autres mélanges de glycérides **caractérisé en ce qu'**il comprend la succession d'étapes suivantes :
- une étape (a) dans laquelle on transestérifie l'huile, la graisse, ou le mélange de glycérides dont l'indice d'acide est au plus de 2 par de l'éthanol dont la teneur en eau est comprise entre 3000 et 5000 ppm en utilisant un catalyseur soluble, ou le devenant au cours de la réaction ;
- une étape (b) de décantation et d'élimination de la glycérine formée ;
- une étape (c) dans laquelle on réalise une deuxième réaction de transestérification de façon à obtenir un produit dont la teneur en esters est d'au moins 97 % en masse ;
- une étape (d) dans laquelle on réalise une évaporation de l'excès d'éthanol en présence du catalyseur, par emploi d'un évaporateur de type film tombant, à une température inférieure à 120°C et un temps de séjour inférieur à 1 minute, l'éthanol obtenu étant alors pratiquement anhydre ;
- une étape (e) dans laquelle l'ester subit une purification par des séquences de lavage à l'eau ; et
- une étape (f) dans laquelle le mélange d'esters est séché sous pression réduite.

2. Procédé selon la revendication 1 **caractérisé en ce que**, dans l'étape (a), le rapport stoechiométrique éthanol/huile est compris entre 1,3 et 2.

3. Procédé selon la revendication 2 **caractérisé en ce que** ledit rapport stoechiométrique éthanol/huile est compris entre 1,6 et 1,8.

4. Procédé selon l'une des revendications 1 à 3 **caractérisé en ce que**, dans l'étape (a) et dans l'étape (c), le catalyseur utilisé est obtenu par dissolution d'une base forte dans de l'alcool, ou encore à partir d'un alcoolate de métal alcalin, ou à partir d'un composé métallique de type alcoolate, alkyle et/ou oxyde.

5. Procédé selon la revendication 4 **caractérisé en ce que** l'on utilise comme catalyseur du méthylate de sodium en solution dans du méthanol.

6. Procédé selon l'une des revendications 1 à 5 **caractérisé en ce que**, dans l'étape (a) et dans l'étape (c), la température de réaction est comprise entre 20 et 100°C.

7. Procédé selon l'une des revendications 1 à 6 **caractérisé en ce que**, dans l'étape (a), le temps de réaction en mode batch qui permet d'atteindre l'équilibre thermodynamique est compris entre 40 et 160 minutes.

8. Procédé selon la l'une des revendications 1 à 7 **caractérisé en ce que**, dans l'étape (b), la décantation naturelle de la glycérine est obtenue à une température du milieu comprise entre 40 et 60°C.

9. Procédé selon l'une des revendications 1 à 8 **caractérisé en ce que**, dans l'étape (c), la quantité d'éthanol utilisée correspond à un rapport stoechiométrique éthanol/huile de départ compris entre 0,3 et 1.

10. Procédé selon la revendication 9 **caractérisé en ce que** ledit rapport stoechiométrique éthanol/huile de départ est compris entre 0,5 et 0,7.

11. Procédé selon l'une des revendications 9 et 10 **caractérisé en ce que**, dans l'étape (c), le temps de catalyse est compris entre 20 et 45 minutes.

12. Procédé selon l'une des revendications 1 à 11 **caractérisé en ce que**, dans l'étape (d), la température est inférieure à 100°C et le temps de séjour est inférieur à 30 secondes.

13. Procédé selon l'une des revendications 1 à 12 **caractérisé en ce que**, dans l'étape (e), on effectue un premier lavage neutralisant au moyen d'un acide minéral fort, puis un ou plusieurs lavage(s) à l'eau pure.

## Patentansprüche

1. Verfahren zur Herstellung von Fettsäureethylestern aus pflanzlichen oder tierischen Ölen oder Fetten oder sonstigen Glyceridmischungen, **dadurch gekennzeichnet, dass** es die Abfolge der folgenden Schritte umfasst:
- einen Schritt (a), in welchem das Öl, das Fett oder die Glyceridmischung, dessen/deren Säurezahl höchstens 2 beträgt, mittels Ethanol, dessen Wassergehalt im Bereich von 3000 bis 5000 ppm liegt, umgeestert wird, wobei ein Katalysator verwendet wird, der löslich ist oder es im Laufe der Reaktion wird;
- einen Schritt (b) des Absetzenlassens und Entfernens des gebildeten Glycerins;
- einen Schritt (c), in welchem eine zweite Umesterungsreaktion derart durchgeführt wird, dass ein Produkt erhalten wird, dessen Gehalt an Estern mindestens 97 Massen-% beträgt;
- einen Schritt (d), in welchem der Überschuss an Ethanol in Gegenwart des Katalysators dadurch verdampft wird, dass ein Fallfilmverdampfer eingesetzt wird, bei einer Temperatur von weniger als 120 °C und einer Verweilzeit von weniger als 1 Minute, wobei der dabei erhaltene Ethanol praktisch wasserfrei ist;
- einen Schritt (e), in welchem der Ester eine Aufreinigung erfährt, indem er mehrmals nacheinander mit Wasser gewaschen wird; und
- einen Schritt (f), in welchem die Mischung aus Estern unter vermindertem Druck getrocknet wird.

2. Verfahren nach Anspruch 1, **dadurch gekennzeichnet, dass** in Schritt (a) das stöchiometrische Ethanol/Öl-Verhältnis im Bereich von 1,3 bis 2 liegt.

3. Verfahren nach Anspruch 2, **dadurch gekennzeichnet, dass** das stöchiometrische Ethanol/Öl-Verhältnis im Bereich von 1,6 bis 1,8 liegt

4. Verfahren nach einem der Ansprüche 1 bis 3, **dadurch gekennzeichnet, dass** der in Schritt (a) und in Schritt (c) verwendete Katalysator erhalten wird, indem eine starke Base in Alkohol aufgelöst wird, oder auch ausgehend von einem Alkalimetallalkoholat, oder ausgehend von einer Metallverbindung des Typs Alkoholat, Alkyl und/oder Oxid.

5. Verfahren nach Anspruch 4, **dadurch gekennzeichnet, dass** als Katalysator Natriummethanolat verwendet wird, welches in Methanol gelöst ist.

6. Verfahren nach einem der Ansprüche 1 bis 5, **dadurch gekennzeichnet, dass** in Schritt (a) und in Schritt (c) die Reaktionstemperatur im Bereich von 20 bis 100 °C liegt.

7. Verfahren nach einem der Ansprüche 1 bis 6, **dadurch gekennzeichnet, dass** in Schritt (a) die Reaktionszeit im diskontinuierlichen Betrieb, welche es ermöglicht, das thermodynamische Gleichgewicht zu erreichen, im Bereich von 40 bis 160 Minuten liegt.

8. Verfahren nach einem der Ansprüche 1 bis 7, **dadurch gekennzeichnet, dass** in Schritt (b) der natürliche Absetzvorgang des Glycerins bei einer Temperatur des Mediums erzielt wird, die im Bereich von 40 bis 60 °C liegt.

9. Verfahren nach einem der Ansprüche 1 bis 8, **dadurch gekennzeichnet, dass** in Schritt (c) die Menge des verwendeten Ethanols einem stöchiometrischen Ethanol/Öl-Ausgangsverhältnis im Bereich von 0,3 bis 1 entspricht.

10. Verfahren nach Anspruch 9, **dadurch gekennzeichnet, dass** das stöchiometrische Ethanol/Öl-Ausgangsverhältnis im Bereich von 0,5 bis 0,7 liegt

11. Verfahren nach einem der Ansprüche 9 und 10, **dadurch gekennzeichnet, dass** in Schritt (c) die Katalysezeit im Bereich von 20 bis 45 Minuten liegt.

12. Verfahren nach einem der Ansprüche 1 bis 11, **dadurch gekennzeichnet, dass** in Schritt (d) die Temperatur niedriger als 100 °C ist und die Verweilzeit geringer als 30 Sekunden ist.

13. Verfahren nach einem der Ansprüche 1 bis 12, **dadurch gekennzeichnet, dass** in Schritt (e) eine erste Waschung durchgeführt wird, wobei eine Neutralisierung mittels einer starken Mineralsäure erfolgt, und dann eine oder mehrere Waschung(en) mit reinem Wasser.

## Claims

1. A method for manufacturing fatty acid ethyl esters from vegetable or animal oils or fat or from other glyceride mixtures, **characterized in that** it comprises the succession of stages as follows:
- a stage (a) wherein the oil, the fat or the glyceride mixture whose acid number is at most 2 is transesterified by ethanol whose water content is comprised in the range 3000 ppm to 5000 ppm using a soluble catalyst or a catalyst that becomes soluble during the reaction,
- a stage (b) wherein the glycerin formed is decanted and removed,
- a stage (c) wherein a second transesterification reaction is carried out so as to obtain a product whose ester content is at least 97 % by mass,
- a stage (d) wherein evaporation of the excess ethanol is carried out in the pretence of the catalyst using a falling film type evaporator, at a temperature below 120°C and with a residence time below 1 minute, the ethanol obtained being then practically anhydrous,
- a stage (e) wherein the ester undergoes purification by means of water wash sequences, and
- a stage (t) wherein the ester mixture is dried under reduced pressure.

2. A method as claimed in claim 1, **characterized in that**, in stage (a), the ethanol/oil stoichiometric ratio ranges between 1,3 and 2,

3. A method as claimed in claim 2, **characterized in that** said ethanol/oil stoichiometric ratio ranges between 1.6 and 1.8.

4. A method as claimed in one of claims 1 to 3, **characterized in that**, in stages (a) and (c), the catalyst used is obtained by dissolution of a strong base in alcohol, or from an alkaline metal alcoholate, or from a metallic compound of alcoholate, alkyl and/or oxide type.

5. A method as claimed in claim 4, **characterized in that** sodium methylate in solution in methanol is used as the catalyst.

6. A method as claimed in one of claims 1 to 5, **characterized in that**, in stages (a) and (c), the reaction temperature ranges between 20°C and 100°C.

7. A method as claimed in one of claims 1 to 6, **characterized in that**, in stage (a), the reaction time in batch mode allowing to reach thermodynamic equilibrium ranges between 40 and 160 minutes.

8. A method as claimed in one of claims 1 to 7, **characterized in that**, in stage (b), natural decantation of the glycerin is obtained at a medium temperature ranging between 40°C and 60°C.

9. A method as claimed in one of claims 1 to 8, **characterized in that**, in stage (c), the amount of ethanol used corresponds to an initial ethanol/oil stoichiometric ratio ranging between 0.3 and 1.

10. A method as claimed in claim 9, **characterized in that** said initial ethanol/oil stoichiometric ratio ranges between 0.5 and 0.7.

11. A method as claimed in any one of claims 9 and 10, **characterized in that**, in stage (c), the catalysis time ranges between 20 and 45 minutes.

12. A method as claimed in one of claims 1 to 11, **characterized in that**, in stage (d), the temperature is below 100°C and the residence time is below 30 seconds.

13. A method as claimed in one of claims 1 to 13, **characterized in that**, in stage (e), a first neutralizing wash is carried out using a strong mineral acid, followed by one or more wash cycles with pure water.
